# EUROPEAN PATENT APPLICATION

(11) **EP 4 830 665 A1**
(43) Date of publication of application: **28.10.2026**
(21) Application number: 24908246.2
(22) Date of filing: 18.11.2024
(51) Int. Cl.: A61F 2/00

(54) **IMPLANTABLE URETHRAL SUPPORT DEVICE FOR TREATING URINARY INCONTINENCE**

(30) Priority: 20.12.2023 KR 20230187143
(71) Applicant: Medielle Inc., Daejeon 34900 (KR)
(72) Inventor: YI, Jeong Yoon, Daejeon 34032 (KR); KOO, Bon Aa, Daejeon 34032 (KR); YI, Su Ho, Daejeon 34032 (KR); YI, Hwa Jeong, Daejeon 34032 (KR)
(74) Representative: Zoli, Filippo
(86) International application number: PCT/KR2024/096618
(87) International publication number: WO 2025/135964

(57) **Abstract**

The present invention relates to an implantable urethral support device for treatment of urinary incontinence. More particularly, the present invention relates to a urethral support device configured such that two left and right fixation body portions spaced apart from each other are arranged to define an angle, and the two fixation body portions are interconnected through two regions, including an upper urethral support-connecting portion and a lower urethral support-connecting portion, so as to be stably seated against posterior regions of the left and right pubic bones forming a pubic symphysis. When downward abdominal pressure is applied, the fixation body portions and the lower urethral support-connecting portion are supported by the pubic bones and resist the downward pressure, while the descending urethra is supported by the upper urethral support-connecting portion, thereby producing bending above the upper urethral support-connecting portion and enabling rapid urethral closure to prevent urinary incontinence.

## Description

### TECHNICAL FIELD

The present invention relates to an implantable urethral support device for treatment of urinary incontinence. More particularly, the present invention relates to a configuration in which two left and right fixation body portions, spaced apart from each other, are arranged to define an angle with respect to one another, and the two fixation body portions are interconnected at two regions, including an upper urethral support-connecting portion and a lower urethral support-connecting portion, so that the device can be stably seated against posterior regions of the left and right pubic bones forming a pubic symphysis.

The invention further relates to a urethral support device for treatment of urinary incontinence in which, when downward abdominal pressure is applied, the fixation body portions and the lower urethral support-connecting portion are supported by the pubic bones and resist the downward pressure, while a descending urethra is supported by the upper urethral support-connecting portion, thereby producing bending above the upper urethral support-connecting portion and enabling rapid urethral closure to prevent urinary incontinence.

### BACKGROUND ART

Urinary incontinence refers to involuntary urine leakage occurring when abdominal pressure suddenly increases, for example during exercise, laughing, or coughing, and is caused by weakening of pelvic floor muscles supporting the pelvic floor and ligaments supporting the urethra.

Causes include aging, pregnancy, childbirth, chronic cough, asthma, and strenuous exercise, and the condition may be aggravated by obesity or menopause.

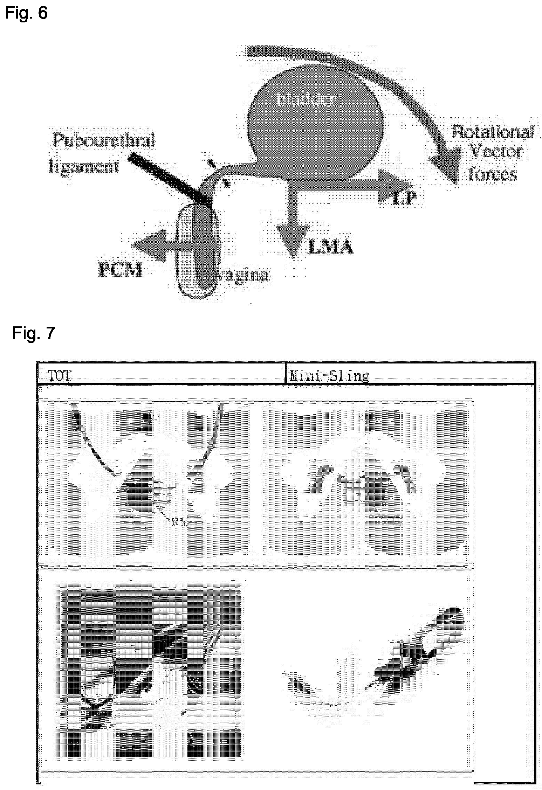

### Referring to FIG. 6,

The pelvic floor muscles are various muscles, including PCM, LMA, and LP, that support female pelvic organs and constitute the pelvic floor. When the pelvic floor muscles are weakened, they are in a relaxed state and are easily displaced downward when abdominal pressure increases during coughing or exercise.

The downwardly displaced pelvic floor muscles pull the bladder and the urethra posteriorly and downwardly, and this force acts as a force that opens the urethra.

A weakened pubourethral ligament has reduced resistance to this force and becomes lax. Therefore, although posterior and downward movement of the bladder and urethra should induce bending of the urethra above the urethral support portion due to the supporting force of the ligament and thereby cause urethral closure, such closure does not occur sufficiently, resulting in urinary incontinence.

This mechanism differs from conventional theories that regarded urinary incontinence as being caused simply by a pressure difference between the bladder and the urethra, and corresponds to the integral theory, which is currently widely accepted.

Based on this understanding, non-surgical methods such as Kegel exercises treat urinary incontinence by strengthening weakened pelvic floor muscles, thereby increasing resistance to abdominal pressure and reducing downward sagging or displacement of the pelvic floor muscles.

As a surgical method, supporting the urethra by fixing an artificial tape to a body structure instead of a weakened ligament is currently a major method for urinary incontinence surgery. For this reason, various surgical procedures have been developed in which an artificial tape is fixed to the obturator fascia, which is located in the obturator foramen of the pelvis and provides strong fixation near the urethra, thereby supporting the urethra.

A urethra supported by an artificial tape inserted in various ways and fixed to the obturator fascia can resist forces generated by the pelvic floor muscles so that the urethra is not opened. By opposing the force that pulls the bladder and urethra posteriorly and downwardly, the tape induces bending of the urethra above the artificial tape and thereby causes urethral closure.

As shown in FIG. 7,
a recent trans-obturator tape (TOT) procedure for treating urinary incontinence involves making groin and suburethral incisions and then forming a passage from the groin through the obturator foramen to the suburethral region using a guide needle holder.

Thereafter, a prolene mesh tape is hooked to the holder under the urethra, or in the reverse order, and pulled toward the groin, thereby fixing the tape to the obturator fascia and supporting the lower portion of the urethra to treat urinary incontinence. The tape is cut under the skin so as not to be exposed beneath the skin or vaginal mucosa, and the skin and suburethral mucosa are sutured.

In a minisling procedure, which is referred to as a single-incision procedure because only a suburethral incision is made, in contrast to the TOT procedure involving suburethral and groin incisions, the tape is inserted and fixed directly from the suburethral region to the obturator fascia using a guide needle holder after the suburethral incision.

However, although such recent procedures are among the least invasive procedures currently available, they generally require spinal anesthesia or sedation, and muscle, ligament, and vascular injuries frequently occur during formation of a passage from the groin through the obturator foramen to the suburethral region using an instrument.

In addition, after surgery, the artificial tape adheres to body tissue, making reoperation difficult and making complete removal of the inserted tape impossible or highly impractical.

Korean Patent Registration No. 10-0947552, registered on March 8, 2010, hereinafter referred to as Prior Art Document 1, discloses a pressure-conversion tension device for treatment of urinary incontinence.

Prior Art Document 1 provides a structure in which elastic portions at both ends are inserted into Retzius spaces formed on both sides of an upper portion of the urethra to fix their positions, and a support portion formed between the two elastic portions is provided as a bag located below the urethra. When the Retzius spaces are reduced due to increased abdominal pressure, the volumes of the elastic portions decrease, an internal flowable material is concentrated in the support portion, and the support portion expands to compress the urethra.

Korean Patent Registration No. 10-1063358, registered on September 1, 2011, hereinafter referred to as Prior Art Document 2, discloses a sub-obturator tension-conversion device.

Prior Art Document 2 provides a structure in which Retzius-space placement portions are inserted into Retzius spaces formed on both sides of the urethra to fix their positions, so that, when abdominal pressure acts, a urethral support shape portion rotates about the two Retzius-space placement portions and compresses the urethra.

Korean Patent Registration No. 10-1461492, registered on November 7, 2014, hereinafter referred to as Prior Art Document 3, discloses a urethral compression tension device for treatment of urinary incontinence.

Prior Art Document 3 is configured with compression portions disposed on both sides or above the urethra and a connecting portion passing below the urethra and connecting the two compression portions, so that, when abdominal pressure acts, the connecting portion rotates about the compression portions and presses the lower portion of the urethra, thereby producing urethral closure.

Prior Art Documents 1 to 3 all use the Retzius space, which is a space formed between the pelvic bone and the bladder by elastic tissue around the bladder and urethra.

The Retzius space has been known to reduce friction between the bladder and surrounding parts and, when abdominal pressure increases, to allow abdominal pressure to be directly transmitted to the urethra through the Retzius space without pressure attenuation by tissue, thereby increasing urethral closure pressure and suppressing urinary incontinence.

However, now that urinary incontinence is understood not as a problem caused by a difference between bladder pressure and urethral pressure resulting from increased bladder pressure, but as urine leakage caused by failure of urethral bending and consequent urethral closure due to weakened pelvic floor muscles and urethral support ligaments, the significance of such a Retzius-space-based concept has diminished.

The Retzius space significantly varies among individuals and, due to the spatial limitations of an elastic tissue-formed space, allows a urethral support device to rotate easily. However, because downward support force due to tissue elasticity is insufficient, the device is displaced downward while rotating, and a constant rotational radius centered on the Retzius space is not formed.

In addition, due to displacement of the urethra and surrounding tissues, the device may not be placed in the correct position but may be obliquely arranged or twisted. As a result, the rotational axis is displaced, making it difficult to accurately support the urethra each time abdominal pressure acts, and an unintended gap may be formed between the device and the urethra, causing urine leakage.

For these reasons, when abdominal pressure is not converted through the device into a supporting force that uniformly supports the urethra, the inserted device may physically damage the vaginal mucosa and may be expelled outside the body.

Accordingly, there is a need for a new structure that can be inserted through a simple procedure and can stably support the urethra when abdominal pressure is generated, thereby solving urinary incontinence.

### PRIOR ART DOCUMENTS

### Patent Documents

Patent Document 1: Korean Patent Registration No. 10-0947552, registered on March 8, 2010, entitled Pressure-Conversion Tension Device for Treatment of Urinary Incontinence.
Patent Document 2: Korean Patent Registration No. 10-1063358, registered on September 1, 2011, entitled Sub-Obturator Tension-Conversion Device.
Patent Document 3: Korean Patent Registration No. 10-1461492, registered on November 7, 2014, entitled Urethral Compression Tension Device for Treatment of Urinary Incontinence.

### SUMMARY OF THE INVENTION

### Technical Problem

The urethral support device for treatment of urinary incontinence according to the present invention is intended to solve the above-described problems of the prior art.

An object of the present invention is to provide a device having a structure that is seated against posterior regions of the left and right pubic bones forming a pubic symphysis, provides direct supporting force against downward abdominal pressure through the pubic bones, and minimizes twisting and tissue injury caused by surrounding tissue.

Another object of the present invention is to provide a device having a structure that can be disposed close to the urethra, thereby rapidly supporting the urethra when abdominal pressure is generated and inducing urethral closure to suppress urinary incontinence.

### Solution to Problem

A urethral support device for treatment of urinary incontinence according to the present invention for solving the above problems is as follows.

In an implantable urethral support device configured to be implanted in a body and to support and close a urethra descending under abdominal pressure, the device includes: a pair of fixation body portions disposed on left and right sides of the urethra and spaced apart from each other, the fixation body portions being configured to engage and be seated against posterior regions of the left and right pubic bones forming a pubic symphysis; an upper urethral support-connecting portion interconnecting upper regions of the spaced left and right fixation body portions and supporting a posterior side of the urethra disposed inside the fixation body portions, wherein, when the urethra descends posteriorly and downwardly under abdominal pressure, the upper urethral support-connecting portion supports the urethra so that urethral closure is induced by bending of the urethra; and a lower urethral support-connecting portion supporting the posterior side of the urethra disposed inside the spaced left and right fixation body portions and interconnecting the left and right fixation body portions below the upper urethral support-connecting portion to suppress twisting of the left and right fixation body portions and assist engagement and seating against posterior regions of the pubic bones.

The fixation body portion may protrude anteriorly relative to the upper urethral support-connecting portion and the lower urethral support-connecting portion so as to be stably seated against the posterior region of the pubic bone, and may be directly connected in an upper-lower direction. In addition, the fixation body portion may include lateral extension portions respectively extending laterally from the upper urethral support-connecting portion and the lower urethral support-connecting portion and a vertical connecting portion interconnecting the lateral extension portions. Further, in order to protrude anteriorly and be stably seated against the posterior region of the pubic bone, the fixation body portion may be formed as an expanded surface wider than the upper urethral support-connecting portion and the lower urethral support-connecting portion, or as an expanded surface integrally connected to an auxiliary frame so that an opening is formed therein.

Here, surfaces defined by the lateral extension portions on both sides, or expanded surfaces of the two fixation body portions, may be arranged to define an angle of 45 degrees or more and less than 180 degrees.

In addition, one or more lower urethral support-connecting portions may be provided.

In addition, the upper urethral support-connecting portion and the lower urethral support-connecting portion may be connected to each other in whole or in part, and the connected portion may be formed as a surface so as to support the posterior side of the urethra.

In addition, the upper urethral support-connecting portion may be formed to extend posteriorly through upper ends of the left and right fixation body portions, and the lower urethral support-connecting portion may be formed to extend posteriorly through lower ends of the left and right fixation body portions.

In addition, the upper urethral support-connecting portion may be formed to extend posteriorly through upper connection points spaced a predetermined distance from upper ends of the left and right fixation body portions toward intermediate portions thereof, and the lower urethral support-connecting portion may be formed to extend posteriorly through lower connection points spaced a predetermined distance from lower ends of the left and right fixation body portions toward the intermediate portions thereof.

In addition, the upper urethral support-connecting portion and the lower urethral support-connecting portion may extend posteriorly from the fixation body portions in parallel with each other so as to be horizontally arranged, upwardly arranged, or downwardly arranged, or one or more of them may extend in different directions so that two extending portions are arranged in a crossing, expanding, or tapered configuration.

In addition, the upper urethral support-connecting portion and the lower urethral support-connecting portion may be respectively connected through the same points of the left and right fixation body portions, with the upper urethral support-connecting portion being connected so as to be upwardly arranged and the lower urethral support-connecting portion being connected so as to be downwardly arranged, such that the two portions are arranged to diverge upwardly and downwardly from the same points.

### Advantageous Effects

The urethral support device for treatment of urinary incontinence according to the above solution provides the following effects.

By arranging anterior surfaces of the protruding fixation body portions at an obtuse or acute angle similar to an angle defined by posterior surfaces of the left and right pubic bones, contact with the pubic bones is maximized, the device is easily seated against the posterior pubic bone regions, and the lower urethral support-connecting portion serves as a bridge between the left and right pubic bones and functions as if fixed to the pelvis. Accordingly, the device has strong supporting force against downward pressure generated by abdominal pressure and also provides support against lateral displacement.

Based on this supporting force, the upper urethral support-connecting portion enables bending and closure of the urethra to occur rapidly. The two fixation body portions connected to each other through a plurality of regions prevent the device from twisting due to displacement of surrounding tissue and are stably seated against the posterior pubic bone regions, so that the urethra can be supported consistently and uniformly under abdominal pressure and can be effectively closed to prevent urinary incontinence.

In addition, the structure of the present invention efficiently disperses and uniformly offsets abdominal pressure, thereby minimizing tissue injury caused by uneven pressure applied to the device.

In addition, due to the angled arrangement of the two fixation body portions, the fixation body portions can be advanced further anteriorly than in a configuration in which the front surfaces of the left and right fixation body portions define an inner angle of 180 degrees. Accordingly, the upper urethral support-connecting portion can be disposed closer to the urethra, and the close arrangement of the upper urethral support-connecting portion shortens the time required for urethral bending when abdominal pressure is generated, thereby enabling rapid urethral closure.

In addition, because the urethral support device of the present invention does not require biological integration with body tissue, reoperation can be performed simply and replacement of the device can be facilitated. Further, the device can be securely fixed in position by suturing to adjacent tissue using a suture passing through an opening of an auxiliary frame, thereby providing a useful device that can be used in various procedural methods.

### BRIEF DESCRIPTION OF DRAWINGS

FIGS. 1A to 1D are a perspective view, a front view, a plan view, and a side view illustrating a urethral support device for treatment of urinary incontinence according to a preferred embodiment of the present invention. FIGS. 1E and 1F are perspective views illustrating a urethral support device having a frame-type configuration according to the present invention.
FIGS. 2A and 2B are a horizontal cross-sectional arrangement view and a vertical cross-sectional arrangement view schematically illustrating a state in which the urethral support device according to a preferred embodiment of the present invention is seated against posterior pubic bone regions.
FIG. 3 is a side view illustrating various configurations in which an upper urethral support-connecting portion and a lower urethral support-connecting portion are respectively connected through upper and lower ends of left and right fixation body portions in the urethral support device according to the present invention.
FIG. 4 is a side view illustrating various configurations in which an upper urethral support-connecting portion and a lower urethral support-connecting portion are respectively connected through points spaced a predetermined distance from upper and lower ends of the left and right fixation body portions in the urethral support device according to the present invention.
FIG. 5 is a side view illustrating other configurations of an upper urethral support-connecting portion and a lower urethral support-connecting portion interconnecting the left and right fixation body portions in the urethral support device according to the present invention.
FIG. 6 is a schematic configuration view illustrating movement states of female pelvic organs.
FIG. 7 illustrates installation states and surgical instruments of conventional TOT and mini-sling procedures for treatment of urinary incontinence.

### DETAILED DESCRIPTION OF THE INVENTION

Hereinafter, embodiments of the present invention will be described in detail with reference to the accompanying drawings. The present invention may be modified in various ways and may have various forms. Specific embodiments are illustrated in the drawings and described in detail herein. However, these embodiments are not intended to limit the present invention to the particular disclosed forms, and should be understood as including all modifications, equivalents, and alternatives falling within the spirit and technical scope of the present invention. Like reference numerals are used for like elements in the description of the drawings. In the accompanying drawings, dimensions of structures may be enlarged or reduced for clarity of the present invention.

The terms used in the present application are used only to describe specific embodiments and are not intended to limit the present invention. Singular expressions include plural expressions unless the context clearly indicates otherwise. In the present application, terms such as "comprise," "include," or "have" are intended to specify the presence of stated features, numbers, steps, operations, elements, or combinations thereof, and should not be understood as precluding the presence or addition of one or more other features, numbers, steps, operations, elements, or combinations thereof.

Unless otherwise defined, all terms used herein, including technical or scientific terms, have the same meanings as generally understood by a person having ordinary skill in the art to which the present invention pertains. Terms that are commonly used and defined in dictionaries should be interpreted as having meanings consistent with their meanings in the context of the relevant art, and should not be interpreted in an idealized or overly formal sense unless expressly defined in the present application.

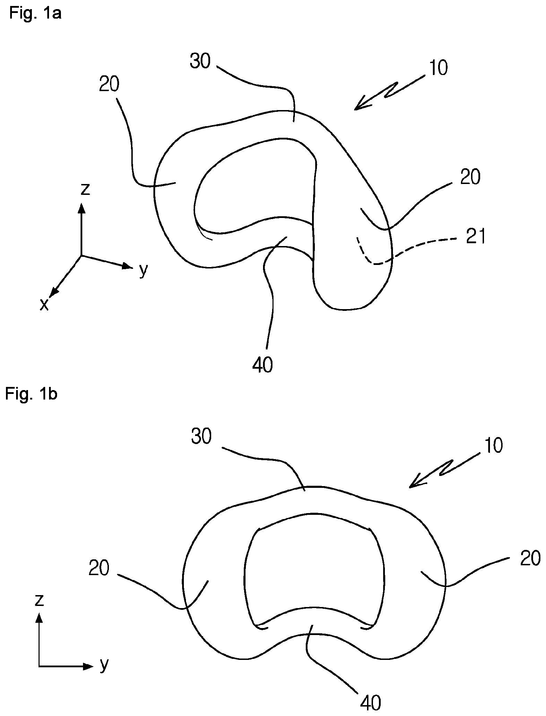

FIGS. 1A to 1D are a perspective view, a front view, a plan view, and a side view illustrating a urethral support device for treatment of urinary incontinence according to a preferred embodiment of the present invention.

As shown, the urethral support device 10 according to the present invention includes a pair of fixation body portions 20 disposed on left and right sides of the urethra and spaced apart from each other, each being seated against a lower posterior pubic bone region, namely a posterior surface on a body-cavity side near the pubic symphysis of the left and right pubic bones constituting the pelvis; an upper urethral support-connecting portion 30 interconnecting upper regions of the spaced left and right fixation body portions and supporting a posterior side of the urethra disposed inside the fixation body portions; and one or more lower urethral support-connecting portions 40 interconnecting the left and right fixation body portions below the upper urethral support-connecting portion and supporting the posterior side of the urethra disposed inside the fixation body portions.

The fixation body portion 20 is an elongated member extending in an upper-lower direction and protruding anteriorly.

The protrusion may be provided in the form of a projection, may be provided by bending an elongated member into an arch shape, and may also include various protruding forms such as a wedge shape.

When the elongated member is bent into an arch shape, it can elastically deform by a certain amount with respect to pressure acting in an upper-lower direction, thereby providing buffering and preventing damage to body tissue due to strong repulsive pressure.

In addition, a protruding anterior surface 21 of the fixation body portion may be expanded to form a surface wider than the upper urethral support-connecting portion or the lower urethral support-connecting portion, and the expansion may be formed as a flat or gently curved surface extending in the left-right width direction.

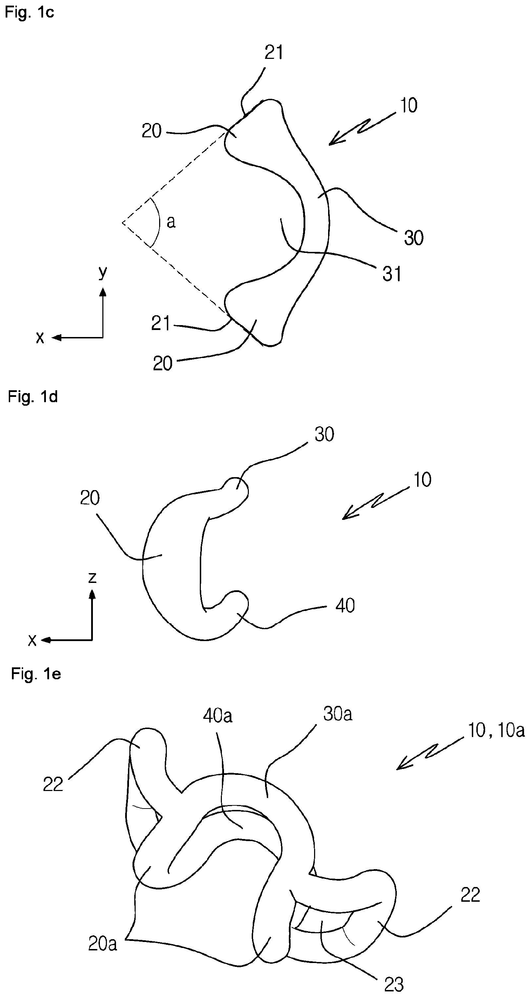

In addition, as shown in FIG. 1E, the fixation body portion may be formed with the same or similar diameter as the upper urethral support-connecting portion and the lower urethral support-connecting portion, and an auxiliary frame may be further connected to a lateral side of the fixation body portion so as to form an expanded surface having an opening therein.

When the fixation body portion is provided as an expanded surface in this manner, the fixation body portion may contact the posterior pubic bone region by line contact or surface contact, thereby enabling stable support.

In addition, since the fixation body portion 20 is inserted into the body, it may be smoothly finished so that no corners are formed, thereby preventing damage to body tissue.

The fixation body portions 20 having such a structure are provided as two portions spaced apart from each other in the left-right direction and are seated in an inner space of the pelvis.

More specifically, the fixation body portions are respectively seated against posterior regions of the pubic bones on both sides of the pubic symphysis constituting the pelvis, particularly posterior regions of the pubic bones on both sides of the subpubic angle near the pubic symphysis, thereby providing fixation in the left-right direction and the upper-lower direction.

The upper urethral support-connecting portion 30 interconnects upper regions of the spaced left and right fixation body portions 20.

Both end portions of the upper urethral support-connecting portion 30 are integrally connected to rear surfaces of the left and right fixation body portions, the fixation body portions being arranged so that their end portions face anteriorly.

As shown in FIG. 1C, the upper urethral support-connecting portion 30 extends and protrudes posteriorly from a rear surface of one fixation body portion, is bent in a U-shape, and is connected to the other fixation body portion.

The upper urethral support-connecting portion 30 has a receiving groove 31 or receiving space formed inside the bend. By placing the urethra in the receiving groove, when abdominal pressure is generated, sagging of the urethra may occur in various directions, including lateral and posterior directions, and the upper urethral support-connecting portion disposed behind the urethra can rapidly support a lower and/or posterior portion of the urethra, thereby inducing urethral closure by bending of the urethra.

Because the receiving groove 31 receives the urethra therein, even if twisting occurs in the urethral support device of the present invention, the device can support the urethra when abdominal pressure is generated and thereby produce urethral closure.

The upper urethral support-connecting portion may be provided in various forms capable of at least partially receiving or surrounding the urethra, in addition to a U-shape, and a surface contacting the urethra may be formed as a curved surface to prevent damage to the urethra during support.

In addition, as shown in FIG. 1D, the upper urethral support-connecting portion 30 may have an intermediate bent portion formed upwardly so as to support a portion of the urethra close to the bladder and thereby induce urethral closure.

Here, the upper urethral support-connecting portion may be formed so that all or part of it protrudes upwardly, horizontally, or downwardly, and may be provided in various forms such as a straight protruding form or a curved protruding form.

When the urethra is closed at a portion close to the bladder in this manner, inflow of urine from the bladder into the urethra can be fundamentally blocked.

In addition, the connection between the upper urethral support-connecting portion 30 and the fixation body portion 20 may be tapered from the wide cross-section of the fixation body portion 20 toward the narrow cross-section of the upper urethral support-connecting portion 30, thereby enabling support of the urethra without urethral damage due to corners or steps.

The lower urethral support-connecting portion 40 is a portion that interconnects the left and right fixation body portions 20 below the upper urethral support-connecting portion 30.

The lower urethral support-connecting portion may also be connected directly and linearly, similar to the upper urethral support-connecting portion, or may be connected while protruding and bending posteriorly in a U-shape, and may be configured to be taperedly connected to the fixation body portions.

Together with the fixation body portions, the lower urethral support-connecting portion increases the contact area at the posterior lower ends of the pubic bones, serves as a bridge between the left and right pubic bones, and functions to be seated and fixed against posterior regions of the pubic bones, thereby strengthening downward support force for the upper urethral support-connecting portion.

Seating against posterior pubic bone regions may also be achieved through the lower urethral support-connecting portion 40. When the lower urethral support-connecting portion 40 protrudes posteriorly and is formed upwardly as shown in FIG. 1D, or when it is formed downwardly, the overall arrangement of the urethral support device may differ when the lower urethral support-connecting portion is seated against the posterior pubic bone region.

For example, as shown in FIG. 1D, when the lower urethral support-connecting portion is formed to protrude in an upper diagonal direction and is installed with the lower urethral support-connecting portion seated against the posterior pubic bone region, an upper side of the fixation body portion may be disposed more posteriorly than a lower side thereof, and the upper urethral support-connecting portion may be provided in a form disposed more posteriorly than the lower urethral support-connecting portion.

In addition, when the lower urethral support-connecting portion is formed to protrude in a lower diagonal direction and is installed with the lower urethral support-connecting portion seated against the posterior pubic bone region, an upper side of the fixation body portion may be disposed more anteriorly than a lower side thereof, and the upper urethral support-connecting portion may be provided in a form disposed more anteriorly than the lower urethral support-connecting portion.

In addition, when the urethral support device 10 of the present invention is mounted upside down, the existing upper urethral support-connecting portion may function as the lower urethral support-connecting portion, and the existing lower urethral support-connecting portion may function as the upper urethral support-connecting portion. Depending on the protruding forms of the upper urethral support-connecting portion and the lower urethral support-connecting portion, the upper urethral support-connecting portion may be disposed forwardly or rearwardly.

When the upper urethral support-connecting portion is disposed forwardly, the upper urethral support-connecting portion can be disposed close to the urethra to provide urethral support even without mounting the urethral support device deeply forwardly from the posterior pubic bone region.

When the upper urethral support-connecting portion is disposed rearwardly, the urethral support device may be brought into close contact with, or be seated against, a wall portion of the pubic bone facing the implanted device, thereby providing stable support against vertically applied pressure.

In addition, the lower urethral support-connecting portion 40 and the upper urethral support-connecting portion 30 may have the same posterior extension length or may have different posterior extension lengths, and may thus be provided in various lengths.

The present invention maintains spacing between the two fixation body portions and minimizes twisting by connecting the rear surfaces of a pair of left and right fixation body portions 20 at two upper and lower regions through the upper urethral support-connecting portion 30 and the lower urethral support-connecting portion 40.

In addition, a plurality of lower urethral support-connecting portions 40 may be formed to increase supporting force of the two fixation body portions and prevent twisting.

In addition, when the upper urethral support-connecting portion and the lower urethral support-connecting portion have the same posterior protruding length, the uppermost portion provides the function of supporting and bending the urethra. However, when any one of a plurality of lower urethral support-connecting portions has a shorter posterior protruding length, the lower urethral support-connecting portion having the shorter length can provide an anti-twisting function while directly supporting or compressing the urethra and increasing urethral closure pressure.

As shown in FIG. 1E, a frame-type urethral support device 10a according to the present invention may be formed by providing a fixation body portion 20a, an upper urethral support-connecting portion 30a, and a lower urethral support-connecting portion 40a, and by further extending an auxiliary frame 22 laterally from the fixation body portion so that an opening 23 is formed. The opening formed by the auxiliary frame may also be formed in a mesh shape to increase structural strength of a portion extending laterally from the fixation body portion.

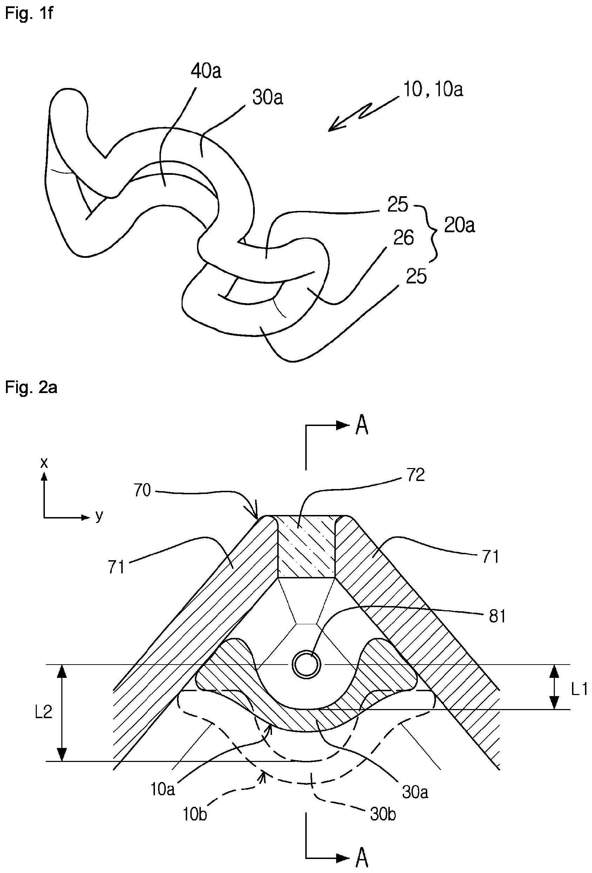

In addition, the frame-type fixation body portion 20a according to the present invention may have, in addition to the form in which the fixation body portion 20a directly and vertically connects the upper urethral support-connecting portion 30a and the lower urethral support-connecting portion 40a as shown in FIG. 1E, a configuration including lateral extension portions 25 respectively extending laterally from end portions of the upper urethral support-connecting portion 30a and the lower urethral support-connecting portion 40a, and a vertical connecting portion 26 interconnecting the upper and lower extension portions, as shown in FIG. 1F.

In addition, an expanded surface formed by the fixation body portion itself as shown in FIG. 1A, a virtual expanded surface formed by the auxiliary frame provided on the lateral side of the fixation body portion as shown in FIG. 1E, or a virtual expanded surface formed by the lateral extension portions respectively extending laterally on both sides as shown in FIG. 1F may be arranged to define an inner angle of 45 degrees or more and less than 180 degrees, so that the device can be advanced and disposed anteriorly when seated against the posterior pubic bone regions described below.

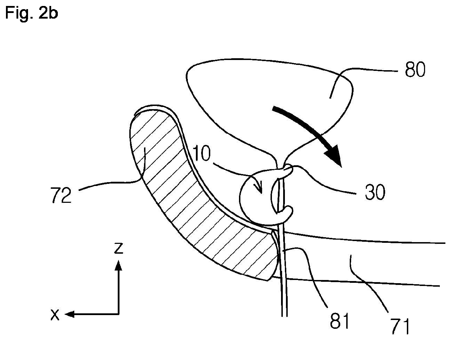

FIGS. 2A and 2B illustrate a state in which the urethral support device according to the present invention is installed at lower posterior pubic bone regions within a body cavity.

In the drawings, the upper side in FIG. 2A and the left side in FIG. 2B correspond to the abdominal side, and the lower side in FIG. 2A and the right side in FIG. 2B correspond to the buttock side. FIG. 2A is a projection view from the head side toward the leg side showing the pubic symphysis and the left and right pubic bones and the device seated against lower posterior surfaces of the pubic bones, and FIG. 2B is a side cross-sectional view.

As shown, in the urethral support device 10 according to the present invention, an inner angle defined by anterior surfaces, which are expanded surfaces of the left and right fixation body portions 20, may be formed within a range of 45 degrees or more and less than 180 degrees.

The fixation body portions 20 are respectively disposed on the left and right sides, and the inner angle defined by the anterior surfaces 21 of the left and right fixation body portions 20 may be formed to be similar to an angle defined by the left and right pubic bones 71 constituting the pelvis 70, so that the urethral support device can be advanced further anteriorly in the posterior pubic bone region, namely in the x-axis direction, and brought into close contact therewith.

This increases contact with the pubic bones, allows the device to be supported by the left and right pubic bones through the lower urethral support-connecting portion, strengthens downward and lateral support, reduces the influence of surrounding tissue, and minimizes deformation of the device due to tissue displacement.

In addition, FIG. 2A illustrates a change in installation position according to the arrangement angle of the left and right fixation body portions 20.

A dashed-line urethral support device 10b in which the inner angle defined by the two anterior surfaces 21, which are expanded surfaces of the left and right fixation body portions, is 180 degrees, and a solid-line urethral support device 10a in which the inner angle of the anterior surfaces of the left and right fixation body portions is 100 degrees, are illustrated.

The solid-line urethral support device 10a and the dashed-line urethral support device 10b are formed identically except for the arrangement angle of the anterior surfaces 21 of the left and right fixation body portions.

As illustrated, because the solid-line urethral support device 10a has a shorter width than the dashed-line urethral support device 10b, the solid-line urethral support device can be advanced further anteriorly, namely upward in the drawing, and seated between the two pubic bones 71 arranged in a triangular-pyramid configuration in the drawing. Therefore, a distance L1 between the upper urethral support-connecting portion 30a of the solid-line urethral support device and the urethra is shorter than a distance L2 between the upper urethral support-connecting portion 30b of the dashed-line urethral support device and the urethra, enabling rapid urethral closure by urethral bending due to close placement.

Of course, when the upper urethral support-connecting portion of the urethral support device is provided in a straight form or in a form protruding anteriorly, it can be positioned closer to the urethra regardless of the arrangement angle of the fixation body portions.

However, to prevent twisting due to tissue pressure inside the body, the urethral support device should be placed close to a pubic-bone wall portion facing the implanted device, namely toward the pubic symphysis side. If the upper urethral support-connecting portion is formed linearly, then when the urethral support device is placed anteriorly, the upper urethral support-connecting portion may press the urethra anteriorly even in a normal state and thereby close the urethra, which may interfere with normal urination.

In addition, to increase the contact area with the urethra and enable efficient urethral bending, it is preferable to form the upper urethral support-connecting portion in a posteriorly protruding and extending form that generally surrounds the urethra, rather than in a straight or anteriorly protruding form.

As shown in FIG. 2A, in the urethral support device 10, it is preferable that the fixation body portions 20 be disposed anteriorly and that the upper urethral support-connecting portion 30 and the lower urethral support-connecting portion 40 be provided in posteriorly protruding U-shaped forms, so that, in a normal state, the urethra 81 disposed posteriorly of the fixation body portions is not compressed, whereas when abdominal pressure is generated, the lower portion of the urethra descending posteriorly is supported, thereby inducing urethral closure by urethral bending.

In addition, as shown in FIG. 2A, because the fixation body portions of the urethral support device according to the present invention are inserted and seated between two pubic bones arranged at an angle of 45 degrees or more and less than 180 degrees, the device can provide supporting force in both lateral directions and thereby be stably fixed in position against lateral pressure.

FIG. 2B schematically illustrates the A-A portion of FIG. 2A. The cross-sectioned portion represents the pubic symphysis connecting both pubic bones, and the urethra is illustrated as passing through a space between both pubic bones near the pubic symphysis.

Here, in the urethral support device 10 according to the present invention, the fixation body portion 20 is seated at a lower end of the pubic symphysis 72 or portions of the left and right pubic bones 71 near the pubic symphysis, and the upper urethral support-connecting portion 30 and the lower urethral support-connecting portion 40 are arranged to surround the posterior side of the urethra 81, namely the right side in the drawing.

Accordingly, the left and right pubic bones 71 are inclined so that an upper width is greater than a lower width, and the fixation body portions 20 of the urethral support device are seated against lower posterior surfaces of the pubic bones by line contact or surface contact and have surfaces expanded upwardly, thereby stably supporting abdominal pressure generated in the upper-lower direction and providing fixation.

In addition, because the fixation body portion 20 is placed close to a pubic-bone wall portion facing the implanted device in the pubic bone 71 having an L-shaped vertical cross-section, it is less affected by varying pressure of body tissue, can minimize deformation, and can maintain its initially installed position as much as possible.

The urethral support device 10 mounted in this manner is disposed so that, in a normal state, the upper urethral support-connecting portion 30 is close to the urethra 81 without compressing the urethra. When abdominal pressure is generated, the bladder 80 moves posteriorly and downwardly, and the urethra 81 connected to the bladder also moves posteriorly and downwardly.

At this time, the upper urethral support-connecting portion 30 of the urethral support device 10 supports the posterior surface of the urethra 81, and the urethra 81 is bent by the upper urethral support-connecting portion 30, thereby producing urethral closure.

In particular, when the upper urethral support-connecting portion 30 is formed to extend upwardly, a portion supporting the urethra 81 can be brought closer to the bladder 80, thereby enabling rapid urethral closure when abdominal pressure is generated.

The urethral support device 10 of the present invention may have various posterior extension forms of the upper urethral support-connecting portion 30 and the lower urethral support-connecting portion 40.

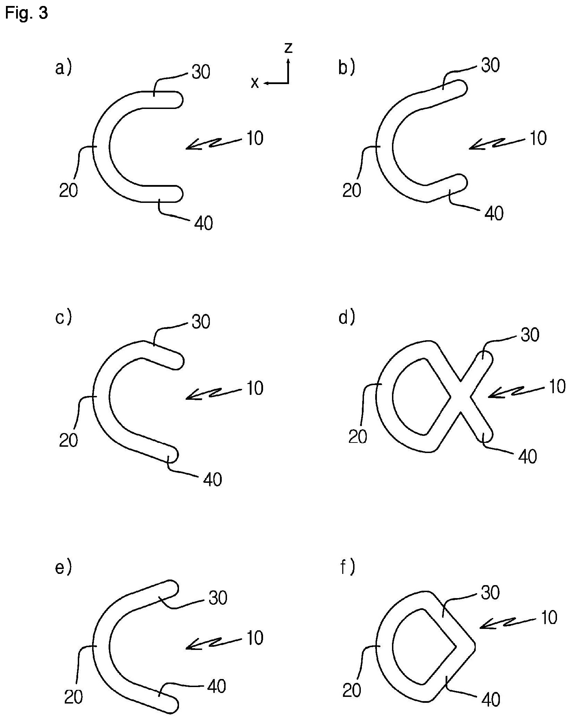

FIG. 3 is a side view of the urethral support device according to the present invention and illustrates various forms in which the upper urethral support-connecting portion 30 and the lower urethral support-connecting portion 40 are connected through upper and lower ends of the left and right fixation body portions 20.

As shown, the upper urethral support-connecting portion 30 and the lower urethral support-connecting portion 40 may be formed by extending and connecting them posteriorly from the upper and lower ends of the fixation body portions 20, and the posterior extension forms of the upper urethral support-connecting portion 30 and the lower urethral support-connecting portion 40 may be parallel, such as extending horizontally posteriorly as in FIG. 3(a), extending upwardly as in FIG. 3(b), or extending downwardly as in FIG. 3(c).

In addition, one or more of the upper urethral support-connecting portion 30 and the lower urethral support-connecting portion 40 may extend in different directions so that the two extending portions cross each other in an upper-lower direction as in FIG. 3(d), diverge from each other in an upper-lower direction as in FIG. 3(e), or are arranged in a tapered configuration toward one point or a center as in FIG. 3(f), thereby preventing twisting of the left and right fixation body portions 20 and enabling positional fixation.

Although the fixation body portion 20 is illustrated as having an arch shape, the configuration is not limited thereto. The upper urethral support-connecting portion 30 may be disposed forwardly and may extend in a diagonal straight form in association with formation of the fixation body portion, and the fixation body portion may also be provided in a curved form corresponding to the posterior pubic bone region.

In addition, the upper urethral support-connecting portion 30 and the lower urethral support-connecting portion 40 may be connected through points spaced apart from the upper and lower ends of the left and right fixation body portions 20.

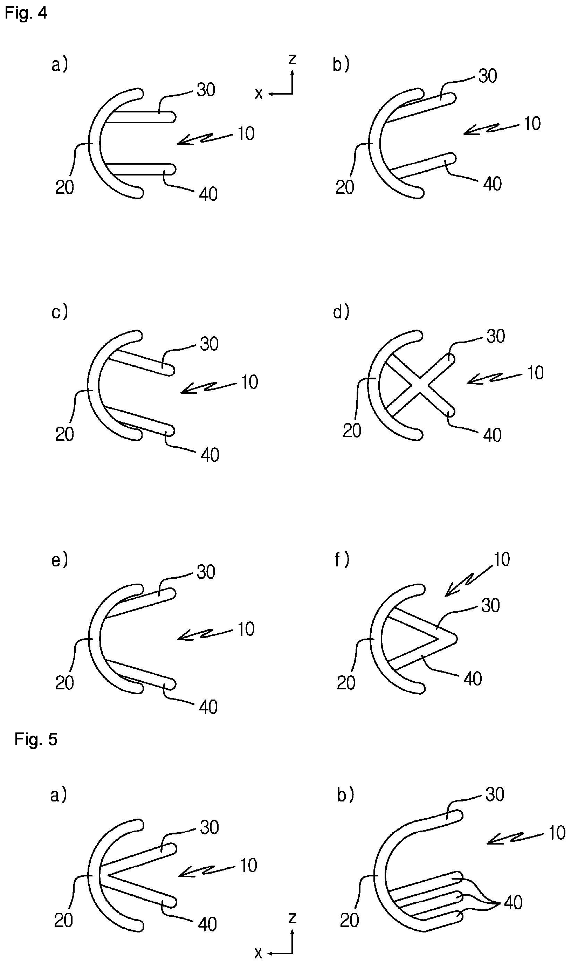

FIG. 4 is a side view of a urethral support device according to another embodiment of the present invention and illustrates various forms in which the upper urethral support-connecting portion 30 and the lower urethral support-connecting portion 40 extend posteriorly and are connected through points spaced a predetermined distance from the upper and lower ends of the left and right fixation body portions 20 toward intermediate portions thereof.

As shown, the upper urethral support-connecting portion 30 and the lower urethral support-connecting portion 40 may be formed by extending and connecting them posteriorly through points spaced a predetermined distance from the upper and lower ends of the fixation body portions 20 toward intermediate portions thereof. The posterior extension forms of the upper urethral support-connecting portion 30 and the lower urethral support-connecting portion 40 may be parallel, such as extending horizontally posteriorly as in FIG. 4(a), extending upwardly as in FIG. 4(b), or extending downwardly as in FIG. 4(c).

In addition, one or more of the upper urethral support-connecting portion 30 and the lower urethral support-connecting portion 40 may extend in different directions so that the two extending portions cross each other in an upper-lower direction as in FIG. 4(d), diverge from each other in an upper-lower direction as in FIG. 4(e), or are arranged in a tapered configuration toward one point or a center as in FIG. 4(f), thereby providing various forms.

As another form, as shown in FIG. 5, the upper urethral support-connecting portion 30 and the lower urethral support-connecting portion 40 may respectively extend from and be connected through a common point at a rear side of each of the left and right fixation body portions 20, with the upper urethral support-connecting portion 30 extending upwardly and the lower urethral support-connecting portion 40 extending downwardly so that they diverge upwardly and downwardly as shown in FIG. 5(a).

When connection is made through the same point in this manner, the anti-twisting effect of the fixation body portion may be somewhat reduced, but using two lines to connect the left and right fixation body portions improves supporting force compared with using only one upper urethral support-connecting portion, and also improves fixation force at the position where the urethral support device is seated.

In addition, as shown in FIG. 5(b), the urethral support device 10 of the present invention may support the left and right fixation body portions 20 by interconnecting them using one upper urethral support-connecting portion 30 and a plurality of lower urethral support-connecting portions 40.

When a plurality of lower urethral support-connecting portions 40 are formed, the left and right fixation body portions are connected at multiple contact points, thereby minimizing twisting, and a shorter lower urethral support-connecting portion may be formed to increase basic urethral closure pressure.

In addition, the plurality of lower urethral support-connecting portions may be provided not only as independent connections as illustrated, but also in a mutually crossing mesh structure.

In addition, the upper urethral support-connecting portion and the lower urethral support-connecting portion may be connected to each other in whole or in part, and the connected portion may be formed as a surface to support the posterior side of the urethra. In this case, the surface may be provided as a wide surface connecting the upper urethral support-connecting portion and the lower urethral support-connecting portion, and may be formed as a wide mesh body to provide both anti-twisting and urethral support functions.

### DESCRIPTION OF REFERENCE NUMERALS

10, 10a: urethral support device
20, 20a: fixation body portion
21: anterior surface
22: auxiliary frame
23: opening
25: lateral extension portion
26: vertical connecting portion
30, 30a: upper urethral support-connecting portion
31: receiving groove
40, 40a: lower urethral support-connecting portion
70: pelvic bone
71: pubic bone
72: pubic symphysis
80: bladder
81: urethra
a: inner angle

## Claims

1. An implantable urethral support device configured to be implanted in a body and to support and close a urethra descending under abdominal pressure, the device comprising:
a pair of fixation body portions disposed on left and right sides of the urethra and spaced apart from each other, the fixation body portions being configured to engage and be seated against posterior pubic bone regions of left and right pubic bones forming a pubic symphysis;
an upper urethral support-connecting portion interconnecting upper regions of the pair of fixation body portions and configured to support a posterior side of the urethra disposed between the pair of fixation body portions, such that, when the urethra descends posteriorly and downwardly under abdominal pressure, the urethra is supported by the upper urethral support-connecting portion and urethral closure is induced by bending of the urethra; and
a lower urethral support-connecting portion disposed below the upper urethral support-connecting portion, interconnecting the pair of fixation body portions, supporting the posterior side of the urethra, suppressing twisting of the pair of fixation body portions, and assisting engagement and seating of the fixation body portions against the posterior pubic bone regions,
wherein each of the fixation body portions is an elongated member extending in an upper-lower direction, protrudes anteriorly relative to the upper urethral support-connecting portion and the lower urethral support-connecting portion, and has a lower portion configured to be seated against the posterior pubic bone region so as to provide fixation against lateral and vertical displacement.

2. The implantable urethral support device of claim 1, wherein each of the fixation body portions protrudes anteriorly and is formed as an expanded surface wider than the upper urethral support-connecting portion and the lower urethral support-connecting portion, or as an expanded surface integrally connected to an auxiliary frame defining an opening therein, so as to stably engage and be seated against the posterior pubic bone region.

3. The implantable urethral support device of claim 2, wherein expanded surfaces of the pair of fixation body portions are arranged to define an angle of 45 degrees or more and less than 180 degrees.

4. The implantable urethral support device of claim 1, wherein each of the fixation body portions includes lateral extension portions respectively extending laterally from the upper urethral support-connecting portion and the lower urethral support-connecting portion, and a vertical connecting portion interconnecting the lateral extension portions.

5. The implantable urethral support device of claim 4, wherein surfaces defined by the lateral extension portions of the pair of fixation body portions are arranged to define an angle of 45 degrees or more and less than 180 degrees.

6. The implantable urethral support device of claim 1, wherein one or more lower urethral support-connecting portions are provided.

7. The implantable urethral support device of claim 1, wherein the upper urethral support-connecting portion and the lower urethral support-connecting portion are connected to each other in whole or in part to form a surface-type support portion, and wherein the surface-type support portion is configured to support the posterior side of the urethra.

8. The implantable urethral support device of claim 1, wherein the upper urethral support-connecting portion is formed to extend posteriorly through upper ends of the pair of fixation body portions, and the lower urethral support-connecting portion is formed to extend posteriorly through lower ends of the pair of fixation body portions,
wherein the upper urethral support-connecting portion and the lower urethral support-connecting portion extend posteriorly from the fixation body portions in parallel with each other to be horizontally arranged, upwardly arranged, or downwardly arranged, or
wherein one or more of the upper urethral support-connecting portion and the lower urethral support-connecting portion extend in different directions so that extending portions thereof are arranged in a crossing, expanding, or tapered configuration.

9. The implantable urethral support device of claim 1, wherein the upper urethral support-connecting portion is formed to extend posteriorly through upper connection points spaced a predetermined distance from upper ends of the pair of fixation body portions toward intermediate portions thereof, and the lower urethral support-connecting portion is formed to extend posteriorly through lower connection points spaced a predetermined distance from lower ends of the pair of fixation body portions toward the intermediate portions thereof,
wherein the upper urethral support-connecting portion and the lower urethral support-connecting portion extend posteriorly from the fixation body portions in parallel with each other to be horizontally arranged, upwardly arranged, or downwardly arranged, or
wherein one or more of the upper urethral support-connecting portion and the lower urethral support-connecting portion extend in different directions so that extending portions thereof are arranged in a crossing, expanding, or tapered configuration.

10. The implantable urethral support device of claim 1, wherein the upper urethral support-connecting portion and the lower urethral support-connecting portion are respectively connected through the same points of the pair of fixation body portions, the upper urethral support-connecting portion being connected so as to be upwardly arranged and the lower urethral support-connecting portion being connected so as to be downwardly arranged, such that the upper urethral support-connecting portion and the lower urethral support-connecting portion are arranged to diverge upwardly and downwardly from the same points.
